# EUROPEAN PATENT SPECIFICATION

(11) **EP 1 887 905 B1**
(45) Date of publication and mention of the grant of the patent: **21.08.2013**
(21) Application number: 06739312.4
(22) Date of filing: 22.03.2006
(51) Int. Cl.: A45C 11/00, B65D 81/22, B65D 85/38

(54) **OPHTHALMIC LENS PACAKGE WITH INTERNAL DRAINAGE MEMBER**
OPHTHALMISCHE LINSENPACKUNG MIT INNEREM DRÄNAGEELEMENT
BOITIER A LENTILLES OPHTALMIQUES DOTE D'UN ELEMENT DE DRAINAGE INTERNE

(30) Priority: 22.03.2005 US 86016
(43) Date of publication of application: 20.02.2008
(73) Proprietor: Johnson & Johnson Vision Care, Inc., Jacksonville, FL 32256 (US)
(72) Inventor: TOKARSKI, Michael, Ponte Vedra, FL 32082 (US); PECK, James, Jacksonville, FL 32224 (US); DZWILL, Edward, Flemington, NJ 08822 (US); BROCK, George, St. Augustine, FL 32092 (US); SMITH, Roger W., Grove City, OH 43123 (US); ULRICH, Michael Scott, Columbus, OH 43204 (US); SANDER, Raymond J., Mt. Vernon, OH 43050 (US)
(74) Representative: Tunstall, Christopher Stephen
(86) International application number: PCT/US2006/010465
(87) International publication number: WO 2006/102450

(56) References cited:
- US-A- 3 621 855
- US-A- 5 431 879
- US-A1- 2004 140 229

## Description

### Field of Invention

The present invention relates to packages for the storage and shipping of ophthalmic lenses, particularly soft contact lenses. More particularly, the present invention relates to packages having an internal drainage system that allows separation of the ophthalmic lens from the solution when the lens is removed from the package.

### Background of the Invention

Soft contact lenses have become increasingly popular since they were first introduced in the 1970's. Due to progressive reductions in manufacturing costs, soft contact lenses are now an attractive and economical choice for the consuming public. Such lenses may be frequently replaced by the consumers (e.g., on a daily or weekly basis) and do not need to be cleaned or sterilized as often as traditional contact lenses. Of course, the consumer desires to keep a supply of lenses on hand that is commensurate with the frequency with which the lenses are replaced. Therefore, consumer demand for disposable soft contact lenses has led to a need for disposable packages that are easy to use and inexpensive. The packages should be constructed to provide safe storage and shipping for the lenses. It is also desirable that the individual packages be as small as possible, so that they may be easily and unobtrusively stored and carried.

Soft contact lenses are often packaged and stored in a hydrated state, which requires that they be sealed in packages with a storage solution. One widely-used package is the "blister pack", which, in general, comprises a rigid plastic container having a flat upper surface with a concave-shaped well that contains a single ophthalmic lens with a quantity of solution. The well is covered with a flexible cover that is sealed along the perimeter of the upper surface. The blister packs are boxed for shipping and storage until a lens is needed by a consumer. The consumer then peels back the flexible cover from the blister pack to expose the ophthalmic lens. The consumer then pours the lens out into his or her hand, together with the saline solution, and places the lens on the tip of his or her finger for application to the eye.

A need remains for ophthalmic lens packaging systems that include a solution for storage and shipping of the ophthalmic lenses, but allow the solution to be separated from the lens at the time that the lens is removed from the package.

In US5431879A, a method is provided for the sterilization or disinfection of an object in need of such treatment.

US2004/0140229A1 discloses a contact lens cleaning and storage case.

In US3621855A, a container is provided with a plural pocketed basket for individually supporting articles, such as contact lenses, within a cleaning and/or storage solution.

### Brief Description of the Drawings

For a more complete understanding of the present invention, reference is made to the following detailed description of the present invention considered in conjunction with the accompanying drawings, in which:
FIG. 1 is a cross-sectional view of an embodiment of a ophthalmic lens package according to the present invention in which the ophthalmic lens package is sealed for shipping and storage.
FIG. 2 is a cross-sectional view of the ophthalmic lens package of FIG. 1 in which the ophthalmic lens package has been opened to provide access to a contact lens.
FIG. 3 is a perspective view of the sealed ophthalmic lens package of FIG. 1.
FIG. 4 is a perspective view of the opened ophthalmic lens package of FIG. 1.
FIG. 5 is a top view of the shell of another embodiment of a ophthalmic lens package according to the present invention.
FIG. 6 is a side view of the ophthalmic lens package of FIG. 5 in which the ophthalmic lens package has been sealed for shipping and storage.
FIG. 7 is a cross-sectional view of the shell of FIG. 5.
FIG. 8 is a frontal view of a plurality of ophthalmic lens packages according to the present invention assembled for display and storage.

### Detailed Description of the invention

An ophthalmic lens package, comprising the features of claim 1.

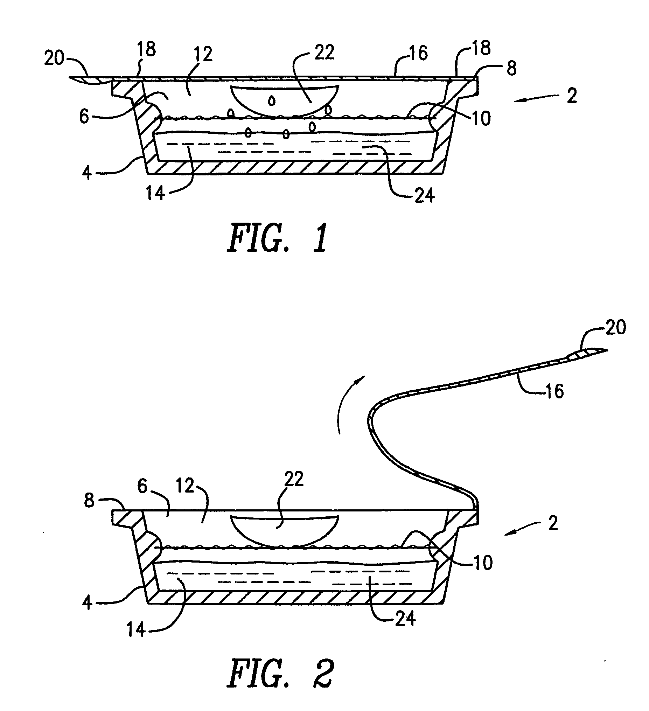

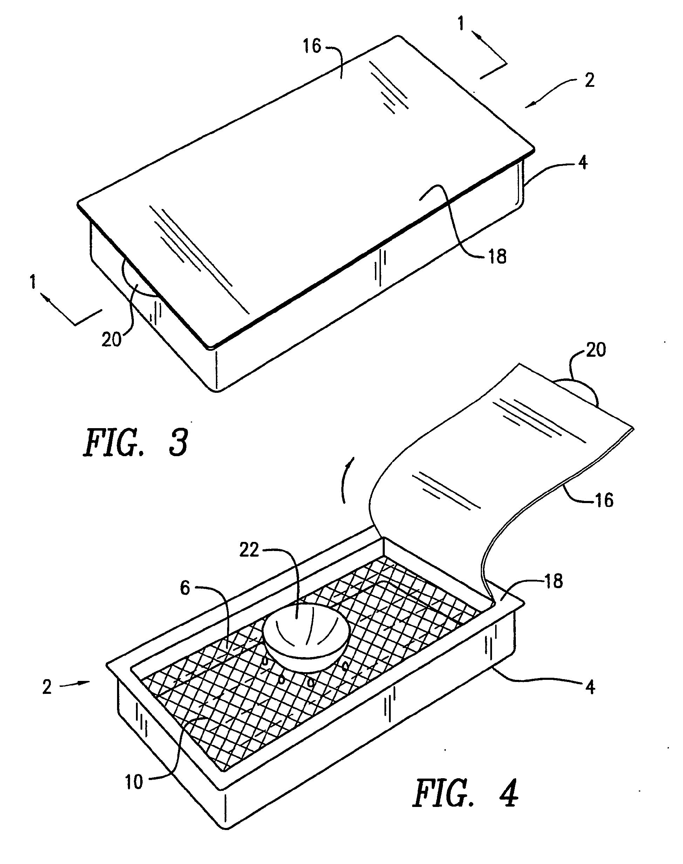

As used herein the term "ophthalmic lens" refers to a device that resides on an eye, including, but not limited to, hard contact lenses, soft contact lenses, intraocular lenses and overlay lenses and preferably soft contact lenses. A first preferred embodiment of an ophthalmic lens package **2** according to the present invention is shown in FIGS. 1-4. Referring to FIGS. 1-4, an ophthalmic lens package **2** has a shell **4** with an interior cavity **6** and an upwardly-facing perimeter rim **8.** A drainage member **10,** such as a mesh or a perforated plastic sheet, is secured to the shell **4** so as to divide the interior cavity **6** into an upper chamber **12** and a lower chamber **14.** A sealing cover **16,** such as a foil member, is used to seal the interior cavity **6** of the shell **4.** A perimeter portion **18** of the sealing cover **16** is secured to the upwardly-facing perimeter rim **8** of the shell **4** so as to form a continuous liquid-tight seal along the entire upwardly-facing perimeter rim **8.** The sealing cover **16** is provided with a tab **20** that extends beyond the perimeter portion **18** of the sealing cover **16** so that the tab **20** may be used as a handle for separating the sealing cover **16** from the shell **4.** The consumer gains access to ophthalmic lens **22** by grasping the tab **20** and pulling it upward and across the upwardly-facing rim **8** of the shell **4,** thereby separating the sealing cover **16** from the shell **4** and exposing ophthalmic lens **22** for retrieval by the consumer (see FIGS. 2 and 4).

The drainage member **10** is arranged within the interior cavity **6** so that ophthalmic lens **22** may rest in an inverted position on the drainage member **10,** within the upper chamber **12,** without contacting the sealing cover **16** that is sealed to the shell **4.** When ophthalmic lens package **2** is prepared for shipping or storage of ophthalmic lens **22,** a volume of saline solution **24** is included within the interior cavity **6** of the shell **4.** The volume of saline solution **24** is less than the volume of the lower chamber **16** so that the entire volume of saline solution **24** may be contained within the lower chamber **16** when ophthalmic lens package 2 is stored in a horizontal position (see, e.g., FIGS. 1 and 2).

When ophthalmic lens container **2** is shipped or stored in a horizontal position, substantially all of the saline solution **24** remains in the lower chamber **14.** Water vapor from the saline solution **24** permeates into the upper chamber **12** through the drainage member **10,** so that ophthalmic lens **22** remains hydrated in the water vapor-saturated atmosphere in the upper chamber **12.** In the event that a portion of the saline solution **24** enters the upper chamber **12,** the saline solution **24** will drain back through the drainage member **10** into the lower chamber **14** when ophthalmic lens package **2** is returned to a horizontal position (see, e.g., FIGS. 1 and 2). Such drainage allows the consumer to remove the lens without inserting his or her finger into standing saline solution. Preferably the drainage leaves ophthalmic lens **22** substantially free of the saline solution **24** when the consumer retrieves ophthalmic lens **22.** Therefore, it is preferable that the drainage member **10** be arranged to allow rapid and complete drainage of the saline solution **24** from the upper chamber **12.** The drainage member **10,** therefore, is preferably an open mesh, although, as an alternative, a perforated member of a hydrophobic plastic material may be used.

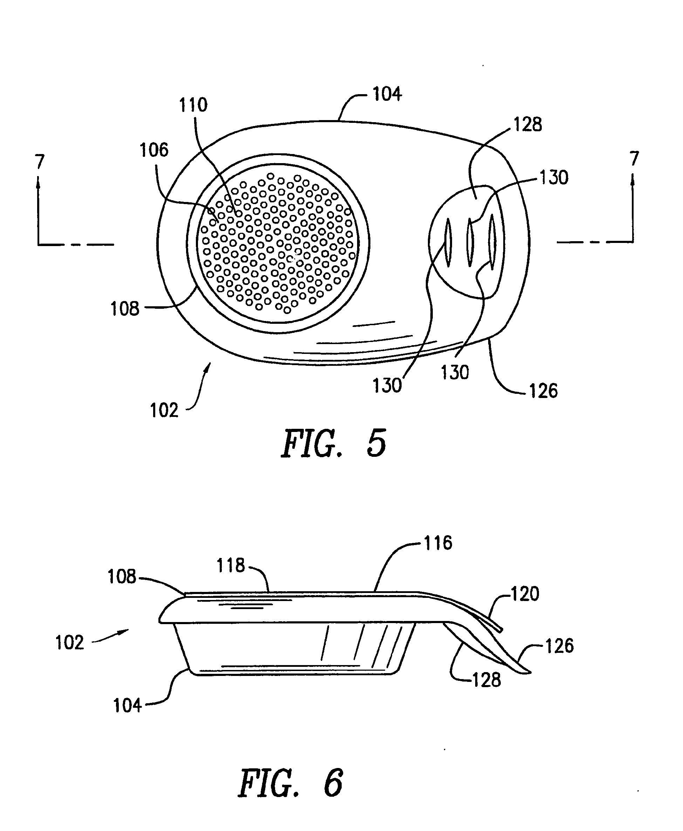

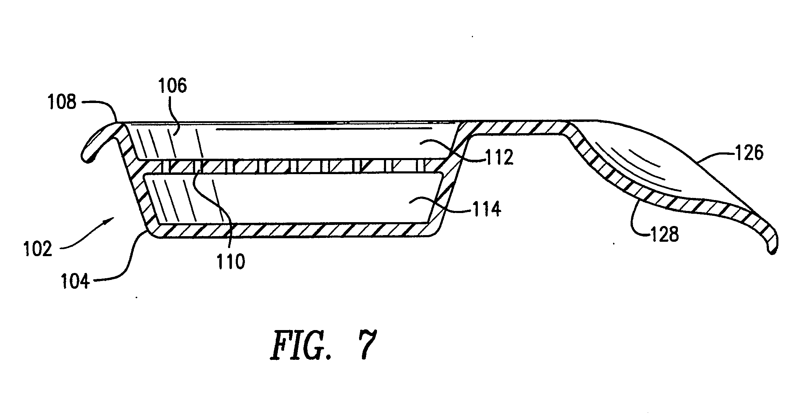

A second preferred embodiment of an ophthalmic lens package 2 according to the present invention is presented in FIGS. 5-7. Reference numbers for features that the second embodiment has in common with the first embodiment are the same as those reference numbers used in the description of the first embodiment, incremented by one hundred (100).

Referring to FIGS. 5-7, an ophthalmic lens package **102** has a shell **104** with an interior cavity **106** and an upwardly-facing perimeter rim **108.** A drainage member **110,** which is, in this second embodiment, a perforated member of a hydrophobic plastic, is molded into the shell **104** and arranged so as to divide the interior cavity **106** into an upper chamber **112** and a lower chamber **114.** A sealing cover **116,** such as a foil member (see FIG. 6), is used to seal the interior cavity **106** of the shell **104.** A perimeter portion **118** of the sealing cover **116** is secured to the upwardly-facing perimeter rim **108** of the shell **104** so as to form a continuous liquid-tight seal along the entire upwardly-facing perimeter rim **108.** The sealing cover **116** is provided with a tab **120** that extends beyond the perimeter rim **108** so that the tab **120** may be used as a handle for separating the sealing cover **116** from the shell **104.** The drainage member **110** is arranged within the interior cavity **106** so that a soft contact lens (not shown) may rest in an inverted position on the drainage member **110,** within the upper chamber **112,** without contacting the sealing cover **116** that is sealed to the shell **104.** When ophthalmic lens package **102** is prepared for shipping or storage of the ophthalmic lens, a volume of saline solution (not shown) is included within the interior cavity **106** of the shell **104.** The volume of saline solution is less than the volume of the lower chamber **114** so that the entire volume of saline solution may be contained within the lower chamber **114** when ophthalmic lens package **102** is stored in a horizontal position. Ophthalmic lens package **102** is further provided with a handle portion **126** which extends from the shell **104** in a direction away from the interior cavity **106.** The handle portion **126** may be provided with a gripping surface, such as a thumb depression **128** with ridges **130.**

The consumer gains access to the ophthalmic lens in a similar manner to that described for the first embodiment (i.e., ophthalmic lens package **2**)**.** The consumer grasps the handle portion **126** between the fingers of one hand and the tab **120** between the fingers of the other hand. The consumer then pulls the tab **120** upward and across the upwardly-facing rim **108** of the shell **104,** thereby separating the sealing cover **116** from the shell **104** and exposing the ophthalmic lens for retrieval by the consumer.

As with the first embodiment, the ophthalmic lens in the second embodiment (i.e., ophthalmic lens package **102**) remains hydrated during shipping and storage in the water vapor-saturated atmosphere in the upper chamber **112** from water vapor that has permeated from saline solution in the lower chamber **114.** Any portion of the saline solution that enters the upper chamber **112** drains through the drainage member **110** into the lower chamber **114** when ophthalmic lens package **102** is returned to a horizontal position, leaving the ophthalmic lens free of the saline solution.

The shell of the invention can be fabricated from a number of know materials, such as polypropylene, polyethylene, and the like. Ophthalmic lens packages **2, 102** of the present invention can be fabricated to have an interior cavity with dimensions of about 2-3 cm by about 2-3 cm and a depth of about 0.5 cm, with its minimum dimensions limited only by the size of the ophthalmic lens to be stored and the volume of saline solution (or other ophthalmic solutions containing ocular medicaments, wetting agents, antimicrobial agents and the like) to be provided. Preferably, the amount of solution is between about 900×10⁻³ml and about 1,600×10⁻³ml, more preferably between about 900×10⁻³ml and about 1,000×10⁻⁵ml. The resulting small overall size of the ophthalmic lens package allows it to be easily and unobtrusively carried. The cover for the shell may be made from foil/polymer laminate or coextrusion, made of a metal layer, such as aluminum and one or more polymer layers, such as polypropylene, coating the metal layer. The cover materials may include any flexible material that acts as a barrier to air borne contaminants and may be hermetically sealed to the shell.

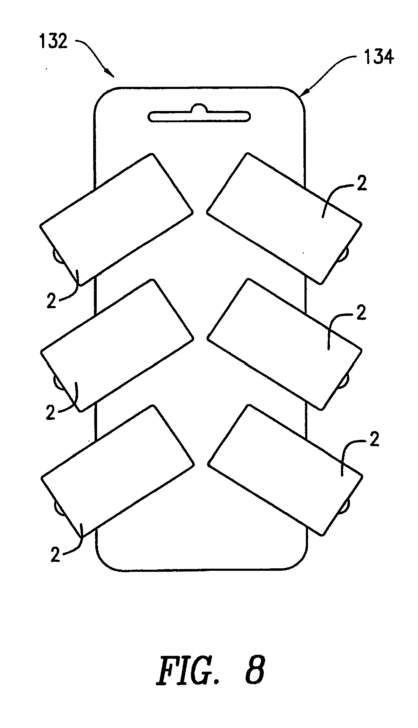

A number of ophthalmic lens packages may also be conveniently stored. For example, FIG. 8 illustrates a storage and display arrangement 132 where six ophthalmic lens packages 2 are mounted on a pegboard card 134 with an overall size of about 12 cm by 18 cm.

## Claims

1. A ophthalmic lens package (2) with aqueous liquid (24), comprising:
a shell (4) defining an opening and having an internal cavity (6) accessible through said opening;
a drainage member (10) permeable to aqueous liquids (24) and arranged within said cavity (6) so as to divide said cavity (6) into an upper chamber (12) and a lower chamber (14), said upper chamber (12) having an interior accessible through said opening and in communication with said lower chamber (14) through said drainage member (10);
a cover (16) extending over said opening and in sealing contact with said shell (4) so as to form a liquid-tight seal with said shell (4);
an ophthalmic lens (22) contained entirely within said upper chamber (12);
and
a quantity of an aqueous liquid (24) contained within said cavity (6), **characterised by** said lower chamber (14) having a volume sufficiently large to contain the entire quantity of said aqueous liquid (24).

2. The package (2) of Claim 1, wherein said drainage member (10) is arranged to allow drainage of aqueous liquid (24) from said upper chamber (12) into said lower chamber (14).

3. The package (2) of Claim 2, wherein said drainage member (10) comprises a mesh.

4. The package (2) of Claim 2, wherein said drainage member (10) comprises a plastic member having perforations therethrough.

5. The package (2) of Claim 1, wherein said shell (4) includes a rim (8) which defines said opening, said package (2) further comprising a cover (16) extending over said opening and forming a liquid-tight seal with said rim (8).

6. The package (2) of Claim 5, wherein said cover (16) includes a foil member having an edge with a grippable tab (20).

7. The package (2) of Claim 1, wherein said opening is defined by a layer of material contiguous with said rim (8), said layer of material extending away from said opening to form a handle (126) that is grippable by a user.

8. The package (2) of Claim 1, further comprising a grippable handle.

## Patentansprüche

1. Augenlinsenpackung (2) mit einer wässerigen Flüssigkeit (24), die Folgendes umfasst:
eine Hülle (4), die eine Öffnung definiert und einen internen Hohlraum (6) aufweist, der durch die Öffnung hindurch zugänglich ist;
ein Ablaufelement (10), das für wässerige Flüssigkeiten (24) durchlässig ist und innerhalb des Hohlraums (6) angeordnet ist, um den Hohlraum (6) in eine obere Kammer (12) und eine untere Kammer (14) zu unterteilen, wobei die obere Kammer (12) ein Inneres aufweist, das durch die Öffnung hindurch zugänglich ist und mit der unteren Kammer (14) durch das Ablaufelement (10) hindurch in Verbindung steht;
eine Abdeckung (16), die sich über der Öffnung erstreckt und mit der Hülle (4) in Abdichtungskontakt steht, um eine flüssigkeitsdichte Abdichtung mit der Hülle (4) zu bilden;
eine Augenlinse (22), die vollständig innerhalb der oberen Kammer (12) enthalten ist; und
eine Menge einer wässerigen Flüssigkeit (24), die innerhalb des Hohlraums (6) enthalten ist, **dadurch gekennzeichnet, dass** die untere Kammer (14) ein Volumen aufweist, das ausreichend groß ist, um die ganze Menge der wässerigen Flüssigkeit (24) zu enthalten.

2. Packung (2) nach Anspruch 1, wobei das Ablaufelement (10) angeordnet ist, um den Ablauf von wässeriger Flüssigkeit (24) von der oberen Kammer (12) in die untere Kammer (14) zu ermöglichen.

3. Packung (2) nach Anspruch 2, wobei das Ablaufelement (10) ein Netz umfasst.

4. Packung (2) nach Anspruch 2, wobei das Ablaufelement (10) ein Kunststoffelement mit Perforationen durch dieses umfasst.

5. Packung (2) nach Anspruch 1, wobei die Hülle (4) einen Rand (8) umfasst, der die Öffnung definiert, wobei die Packung (2) ferner eine Abdeckung (16) umfasst, die sich über der Öffnung erstreckt und eine flüssigkeitsdichte Abdichtung mit dem Rand (8) bildet.

6. Packung (2) nach Anspruch 5, wobei die Abdeckung (16) ein Folienelement mit einer Kante mit einer greifbaren Lasche (20) umfasst.

7. Packung (2) nach Anspruch 1, wobei die Öffnung durch eine Materialschicht definiert ist, die mit dem Rand (8) zusammenhängend ist, wobei sich die Materialschicht von der Öffnung weg erstreckt, um einen Griff (126) zu bilden, der von einem Benutzer greifbar ist.

8. Packung (2) nach Anspruch 1, die ferner einen greifbaren Griff umfasst.

## Revendications

1. Boîtier pour lentilles ophtalmiques (2) avec un liquide aqueux (24), comprenant :
une coque (4) définissant une ouverture et ayant une cavité interne (6) à laquelle on peut accéder à travers ladite ouverture ;
un organe de drainage (10) perméable aux liquides aqueux (24) et agencé dans ladite cavité (6) de manière à diviser ladite cavité (6) en une chambre supérieure (12) et une chambre inférieure (14),
ladite chambre supérieure (12) ayant un intérieur auquel on peut accéder à travers ladite ouverture et communiquant avec ladite chambre inférieure (14) par le biais dudit organe de drainage (10), un couvercle (16) s'étendant par-dessus ladite ouverture et en contact d'étanchéité avec ladite coque (4) de manière à former un joint étanche aux liquides avec ladite coque (4) ;
une lentille ophtalmique (22) contenue entièrement à l'intérieur de ladite chambre supérieure (12), et
une quantité d'un liquide aqueux (24) contenu à l'intérieur de ladite cavité (6), **caractérisé en ce que** ladite chambre inférieure (14) a un volume suffisamment grand pour contenir la quantité totale dudit liquide aqueux (24).

2. Boîtier (2) selon la revendication 1, dans lequel ledit organe de drainage (10) est agencé de manière à permettre le drainage de liquide aqueux (24) depuis ladite chambre supérieure (12) dans ladite chambre inférieure (14).

3. Boîtier (2) selon la revendication 2, dans lequel ledit organe de drainage (10) comprend un treillis.

4. Boîtier (2) selon la revendication 2, dans lequel ledit organe de drainage (10) comprend un organe en plastique percé de perforations.

5. Boîtier (2) selon la revendication 1, dans lequel ladite coque (4) comporte un rebord (8) qui définit ladite ouverture, ledit boîtier (2) comprenant en outre un couvercle (16) s'étendant par-dessus ladite ouverture et formant un joint étanche aux liquides avec ledit rebord (8).

6. Boîtier (2) selon la revendication 5, dans lequel ledit couvercle (16) comporte un organe en film ayant un bord avec une languette de préhension (20).

7. Boîtier (2) selon la revendication 1, dans lequel ladite ouverture est définie par une couche de matériau contigüe audit rebord (8), ladite couche de matériau s'étendant à l'écart de ladite ouverture pour former une poignée (126) qui peut être saisie par un utilisateur.

8. Boîtier (2) selon la revendication 1, comprenant en outre une poignée de préhension.
